(19) Europäisches Patentamt / European Patent Office / Office européen des brevets

(11) **EP 1 918 383 A1**

(12) **EUROPEAN PATENT APPLICATION**

(43) Date of publication:
**07.05.2008 Bulletin 2008/19**

(51) Int Cl.:
***C12P 13/02*** *(2006.01)*

(21) Application number: **06022822.8**

(22) Date of filing: **02.11.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **DSM IP Assets B.V.**
**6411 TE Heerlen (NL)**

(72) Inventor: **Flores-Candia, Juana-Lucia**
**CH-4126 Bettingen (CH)**

(74) Representative: **Keller, Günter et al**
**Lederer & Keller**
**Patentanwälte**
**Prinzregentenstrasse 16**
**80538 München (DE)**

(54) **Semi-continuous fermentation process for pantothenate production**

(57)     The present invention relates to a new semi-continuous process (SCP) for the production of pantothenate by fermentation. This process provides an improved productivity and product concentration while the strain capability of converting glucose into pantothenate maintains stable over a long period of cultivation.

The SCP enhances the pantothenate synthesis up to 41 g/l within 48 h as compared to the ~26.6 g/l achieved with the standard riboflavin process using the same strain (*Bacillus subtilis*, genotype $P_{15}panBCD$ $P_{15}panE$). The semi-continuous fermentation process is based on a continuous feeding rate and a discontinuous medium withdrawal. The new semi-continuous process for the pantothenate production allows longer cultivation times of more than 140 hours for *Bacillus* strains without drastically diminishing specific productivities and glucose conversion efficiency.

**Description**

The present invention relates to a new semi-continuous process (SCP) for the production of pantothenate by fermentation. This process provides an improved productivity and product concentration while the strain capability of converting glucose into pantothenate maintains stable over a long period of cultivation.

Pantothenate is also known as pantothenic acid or vitamin B5 and its calcium form as CalPan. This water soluble vitamin is an essential nutrient for mammals, including livestock and humans. In cells, pantothenate is primarily used for the biosynthesis of coenzyme A (CoA) and acyl carrier protein (ACP). These coenzymes function in the metabolism of acyl moieties which form thioesters with the sulfide group of the 4'-phosphopantheine portion of these molecules. These coenzymes are essential in all cells, participating in over 100 different intermediary reactions in cellular metabolism.

The main route for synthesizing pantothenate is via chemical synthesis from bulk chemicals. The drastic price reductions on the market and the excessive substrate costs for the chemical synthesis as well as the requirements for optical resolution of racemic intermediates lead to an interest in an improved fermentation production of pantothenate.

Different approaches exist to increase the production performance considering that the product yield ($Y_{P/S}$) is normally regarded as strain specific and that the productivity ($P_V$) and production concentration (P) are a result of the operation mode.

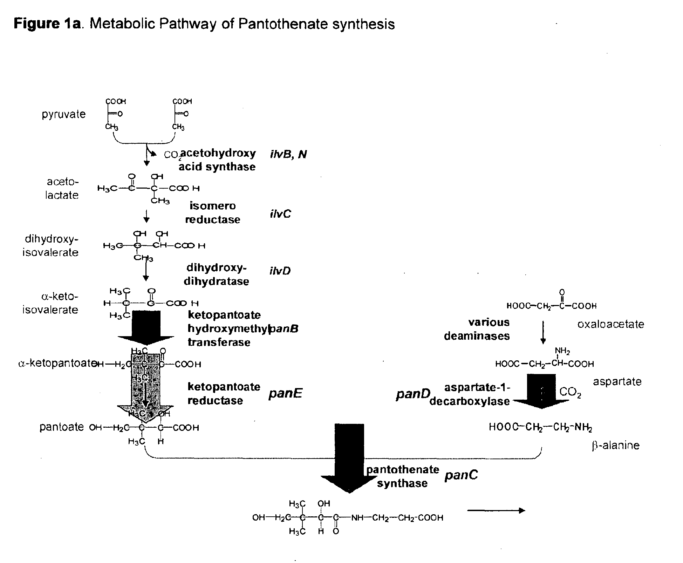

The pantothenate production in bacteria results from the condensation of pantoate and β-alanine and involves the pantothenate biosynthetic enzymes ketopanthoate hydroxylmethyltransferase (the *panB* gene product), ketopanthoate reductase (the *panE* gene product), aspartate-A-decarboxylase (the *panD* gene product) and pantothenate synthetase (the *panC* gene product) as can be seen in Figure 1a.

WO 01/21772 discloses a method for increasing the productivity of a *Bacillus* strain by increasing the expression of the *panB, panC, panD* and *panE* genes and by increasing the expression of theilvBNC and ilvD genes. The resulting strains convert glucose (pyruvate) to commercially attractive quantities of pantothenate.

WO 2004/005525 discloses methods for further enhancing pantothenate production by modulating a biosynthetic pathway that supplies a substrate for the pantothenate biosynthetic pathway, namely the methylene tetrahydrofolate (MTF). In particular, it has been discovered that increasing levels of MTF by modification of the MTF biosynthetic pathway results in enhanced levels of the key pantothenate biosynthetic pathway intermediate, ketopanthoate. Enhanced ketopanthoate levels, in turn, result in significantly enhanced pantothenate production levels in appropriately engineered strains. WO 2004/005525 describes at least three effective means of modifying the MTF biosynthetic pathway.

US 60/262,995 discloses pantothenate production strains that have been engineered to minimize utilization of various pantothenate biosynthetic enzymes and/or isoleucine-valine-biosynthetic enzymes and/or their respective substrates from being used to produce an alternative product identified as 3-(2-hydroxy-3-methyl-butyrylamino)-propionic acid (HMBPA).

The present invention provides a new semi-continuous process for an improved production of pantothenate. In a conventional fed-batch reaction the nutrient or substrate is added in a continuous manner until the maximum filling degree of the reactor is reached. Thus the period of fermentation is restricted by the filling capacity. While chemostat mode operation is able to remove this bottleneck through continuous removal of the fermented media and thereby keeping a constant reaction volume, cell densities are drastically reduced as the dilution rate increases. Whereas high dilution rates are required for high product output, they result in low product concentrations, which in turn reflects the low cell densities. Furthermore, chemostat cultivation of *Bacillus subtilis* for vitamin production (B2, B5), has shown to be instable. In the particular case of pantothenate; both, glucose conversion into product, and productivity were continuously diminishing after 80 h of cultivation time. To overcome these drawbacks an intermediate process was designed, a semi-continuous process, where the restriction of reaction time by reactor capacity is removed by introducing intermittent media withdrawals as the maximum filling degree is reached and high cell densities are maintained by limiting media removal to lower than 20%. High cell density and the possibility of extending the reaction time allow higher product accumulation per volume unit and high reactor turnover.

Starting from a standard fed-batch process as applied for vitamin $B_2$ (in the following $B_2$ process) the examples of the present invention describe step by step how alternative design approaches were introduced in order to develop a cost efficient process for the production of CalPan by *B. subtilis.*

An evaluation of the $B_2$ process suggested that this process design is not optimal for the production of pantothenate. Furthermore, co-feeding experiments and pathway assessment demonstrated that process intensification is feasible. Up to 107 g/l of pantothenate could be produced by combining the supply of pantoate together with β-alanine.

Process intensification efforts were based upon medium reformulation and optimization of the feeding profiles, which resulted in the Calpan Process (CP). Unexpectedly, CP allowed improvements in the fermenter output up to 80% as compared to the reference process ($B_2$ process (US 2005/0124030 A1). Similarly, the titer of the product pantothenate was enhanced from ~26 g/l up to -34 g/l in 48h fermentation time, using a strain with only 4 pan genes overexpressed. Further process improvement focused in extending the fermentation time while maintaining high reaction rates and consequently higher outputs. It was found that a Semi-Continuous Process(SCP) allowed unexpectedly high fermentation times while the strain capability of converting glucose into pantothenate was maintained stable.

For the first time cultivation of *B. subtillis* could be extended from 48h up to 143h resulting in more than two times improvement of the fermenter throughput without diminishing the glucose conversion efficiency as compared to the 48h fed-batch runs. The main benefit of the process of the present invention (SCP) is that the fermenter is kept at its highest productivity for a significant period of time, while in standard fed-batch process it may be down to zero due to turn around operations. These results were also reproduced using the industrial glucose syrup (74 % solid content, 95 % Dextrose equivalent). Microarray analysis showed also that the pan genes overexpression remained at high levels towards the end of the fermentation. SCP has shown also to have the capability of reducing the content of pantothenate related metabolites and hence offers a better broth quality for downstream processing. Finally, an economic evaluation showed that the utilization of the process developed in this work results in a decrease not only in product manufacturing cost but also in the level of capital investment required.

Therefore the present invention refers to a new approach to improve the productivity and product concentration of pantothenate by providing a new semi-continuous process. This technology allows the production of pantothenate by the following design strategies: a) controlled bacterial growth within a range where the conversion of glucose into pantothenate ($Y_{P/S}$) and the specific pantothenate production ($q_{P/S}$) are at an optimum, b) enabling such bacterial growth conditions by modifying the culture medium and applying the appropriate feeding profile, c) applying continuous feeding while performing discontinuous withdrawals, d) withdrawals are restricted to lower than 20% of fermented media to maintain high cell densities.

One strain which can preferably be used for the fermentation process is a genetically modified *Bacillus subtilis,* an overproducer of pantothenate having at least 2 pantothenate genes overexpressed. In fermentation processes the product yield ($Y_{P/S}$) determines the use and cost contribution of the raw materials. Volumetric productivity or reactor throughput has direct input on the production costs and investment. Similarly, high product concentration at the reactor outlet is generally strived for in an attempt to achieve a cost-efficient downstream processing. The present invention provides a new process design to improve production performance indexes set from economic targets. The SC(semi-continuous)-process of the present invention enhances the pantothenate synthesis up to 41 g/l within 48 hours as compared to the ~26.6 g/l achieved with a standard fed-batch process (as used in the riboflavin process) using the same strain. Furthermore, the SC-process allows to reduce the amount of HMBPA, which is formed as a by-product. The product output can additionally be improved by increasing the carbon source compared to the amounts used in normal fed batch processes. In a conservative scenario, the total carbon load is more than two times higher, compared to a fed-batch process.

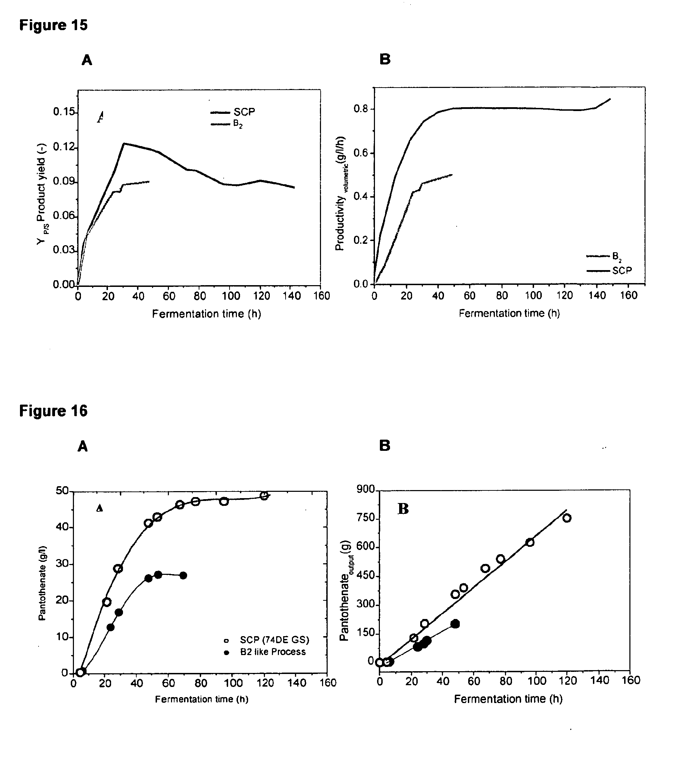

A further object of the present invention is to provide a fermentation process for the pantothenate production, which is effective even when carried out at operation times of more than 100 hours. A major problem of the extended fermentation arises due to the drastically diminishing specific productivity and glucose conversion into pantothenate if the cultivation time exceeds 48 hours which is a typical period of time for a standard fed-batch process. This drawback has been overcome by the present invention by using a novel semi-continuous process, which provides a steady volumetric productivity towards the end of the fermentation, which can be seen in Figure 15.

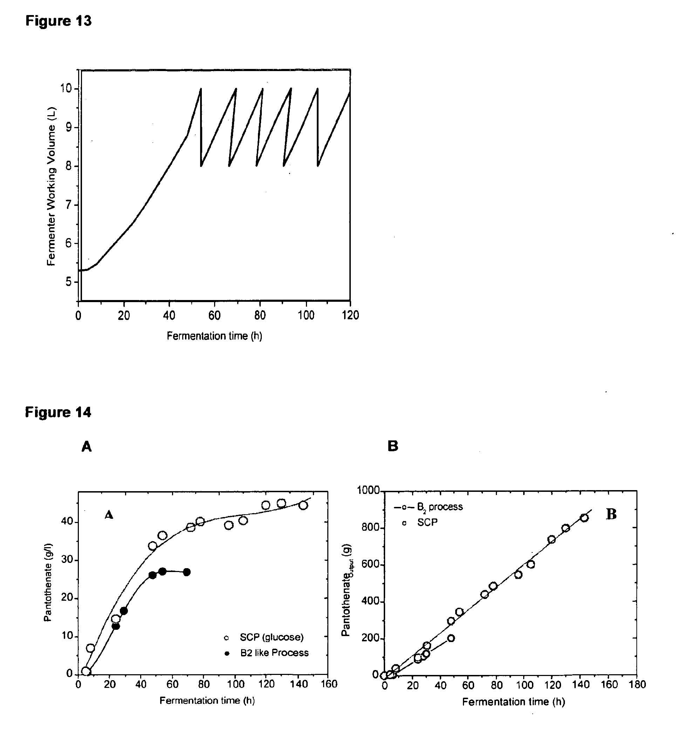

In order to show the improvements on the volumetric productivity achieved by the semi-continuous process according to the invention a comparison to known processes has to be made, wherein exactly the same microorganisms have to be used. Otherwise it is not possible to determine the differences between the volumetric productivity due to the different capability of the different strains and the differences in volumetric productivity due to different operational approaches. Therefore the semi-continuous fermentation process is compared to the standard riboflavine process, using the same strain. The volumetric productivity (g/l/h) as well as the total product output (g) of the process of the present invention could be increased by a factor of 2. Similarly the product concentration could be enhanced from about 26.6 g/l to higher than 41 g/l. The semi-continuous process of the present invention comprises medium withdrawals, performed in specific time intervals to provide a working volume in a specifically selected range. This operation and mode is shown in Figure 13 and is described in more detail in the Examples.

Although the continuous fermentation process (chemostat) is known to allow a higher productivity, it was unexpectedly found that commercial production of pantothenis is cheaper, more efficient and therefore advantageous when using the semi-continuous process according to the present invention.

Reasons for the high yield obtained with the semi-continuous fermentation process are for example the feeding profile according to the invention, the semi-continuous medium withdrawals, the media formulation and the ability to maintain high active cell densities over a long period of time. Whereas a continuous feeding rate can be applied, the medium withdrawals are performed in intervals (discontinuous) in such a manner that for example 10-20% of the medium related to the whole fermenter working volume at the time of the medium withdrawal is at once removed from the reactor. A continuous removal of medium is known in the art (continuous culture). The continuous removal has disadvantages compared to the medium removal executed on intermittent basis in a volume range of 10%-20% of the total working volume.

The present invention therefore provides a method for producing pantothenate by a semi-continuous fermentation process, wherein

  a) the operation time of the process is more than 48 hours, further preferred 60 hours, even further preferred 80 hours and most preferred more than 140 hours;

  b) the feeding is performed at a continuous feeding rate;

  c) the feeding rate and profile are set within a range for the bacterial growth rate ($\mu$) that maximizes yield ($Y_{P/S}$) and specific productivity ($q_{P/X}$).

  d) fermented medium is withdrawn at different times during the fermentation process; and

  e) withdrawn levels are defined by cell densities targets and feeding profile applied whereby preferably each withdrawal of medium comprises to remove 5%-20%, further preferred 10-15%, medium by volume of the fermenter working volume at the time of the medium withdrawal during the fermentation process. Preferably the amount of withdrawn fermented medium is replaced by fresh medium.

In a preferred embodiment of the invention, the range for the bacterial growth rate ($\mu$) is between 0.03 h$^{-1}$ and 0.07 h$^{-1}$.

It is further preferred that the replacement of withdrawn fermented medium by fresh medium is performed by the continuous feeding, and wherein the continuous amount of feeding solution (g/h) is adjusted to replace the amount of withdrawn medium until the next medium withdrawal is performed.

It is also preferred that the medium withdrawal is performed more than 2 times, further preferred 5 times, even further preferred 10 times and most preferred 15 times, during the fermentation process.

In a preferred embodiment of the invention, the medium withdrawal is performed in intervals of less than 30 hours, further preferred less than 24 hours and even further preferred less than 12 hours and most preferred less than 6 hours.

In a further preferred embodiment of the method, the first medium withdrawal is not performed before 12 hours of operation time.

In an especially preferred embodiment, the maximum filling ($V_R^{max.}$) of the fermentation tank is at least 1.5 fold, further preferred 1.1 fold, the volume of the initial starting volume.

It is further preferred that the feeding solution is reformulated after 48 hours to comprise a higher $PO_4^{3-}$ ion concentration.

In a further preferred embodiment of the method, the reformulated feeding solution comprises a concentration of $PO_4^{3-}$ ions to keep the $PO_4^{3-}$ ion concentration in the fermentation medium at a level of 500 mg/l or higher, further preferred at a level of 700 mg/l or higher.

It is even further preferred that the reformulated feeding solution comprises the following ingredients at a minimum concentration of: $KH_2PO_4$, 6 g/l, further preferred 8 g/l; $Na_2HPO_4 \cdot 12H_2O$, 10 g/l, further preferred 14 g/l; $K_2HPO_4$, 6 g/l, further preferred 8 g/l.

In a preferred embodiment of the method, the dissolved oxygen ($pO_2$) level is kept at $\geq 15\%$

An especially preferred embodiment of the invention comprises a continuous feeding rate, wherein the feeding rate ranges from 4 g (glucose) /l/h up to 41 g/l/h.

In a further preferred embodiment, the pantothenate is produced at a level greater than 34 g/l, further preferred 41 g/l, before the first withdrawal of medium is performed.

In another embodiment of the method, the ratio of the initial starting volume in the fermentation tank/ total amount of withdrawn medium is in the range of 1:1 - 1:2, further preferred 1:2-1:4.

The present invention refers to a semi-continuous operation mode for submerged cultivations for the production of pantothenate.

The semi-continuous fermentation process of the present invention uses microorganisms which are pantothenate overproducers. Preferred strains are: *Bacillus, Corynebacterium, Lactobacillus, Lactococci, Streptomyces.* Preferably the microorganism is of the genus *Bacillus.* Preferable strains of the genus *Bacillus* are: *Bacillus subtilis, Bacillus lentimorbus, Bacillus lentus, Bacillus firmus, Bacillus pantothenicus, Bacillus amyloliquefaciens, Bacillus cereus, Bacillus circulans, Bacillus coagulans, Bacillus licheniformis, Bacillus megaterium, Bacillus pumilus, Bacillus thuringiensis, Bacillus halodurans, Bacillus brevis* and *Bacillus stearothermophilus.*

The strain which is especially preferred for the synthesis of pantothenate is *Bacillus subtilis.* It is further preferred that the *Bacillus subtilis* strain overexpresses at least two pantothenate biosynthetic enzymes. It is even further preferred that at least two pantothenate biosynthetic enzymes selected from the group consisting of *panB, panC, panD* are overexpressed. It is especially preferred that the *panB, panC* and *panD* biosynthetic enzymes are overexpressed. It is most preferred to use a *Bacillus subtilis* strain having the genotype $P_{15}panBCD\ P_{15}panE$. This strain is derived from a strain which has the operon $P_{15}panBCD$ as the panE gene overexpressed (e.g. described in WO 01/21772).

The microorganisms can be cultured in liquid medium. The term "culturing" includes maintaining and/or growing a living microorganism of the present invention (e.g., maintaining and/or a culture or strain). The culturing is performed under conditions to improve the productivity and product concentration while maintaining the strain capability of glucose conversion into pantothenate. The parameters like temperature, pressure and pH are analog to fermentation processes known in the art (US 2005/0124030), whereas it is preferred to control the temperature at 30-50°C, further preferred 37-40°C, more preferred at 38.5-39.5°C. Preferably the pH is kept at 6-8, further preferred 6.8 ± 0.5 and even more preferred at 6.8 ± 0.1. In addition it is preferred to control the pH-value by addition of $NH_4OH$ 27%.

The specific parameters for the semi-continuous process according to the present invention are described in more detail below.

The semi-continuous process can be used for operation times of more than 48 hours, preferably more than 100 hours, even more preferred for more than 120 hours and most preferred for more than 140 hours. The operation time in this context is defined as the fermentation time in the main fermentation reactor.

The semi-continuous fermentation process can be carried out in any containment such as stirred tank reactors or any known containment suitable for culturing. The containment can be chosen depending on the scale of the process. Additionally the containment should be suitable for providing a continuous feeding rate and allowing a discontinuous medium withdrawal and the necessary stirring or shaking of the culture. The size of the containments depends on the scale of the pantothenate production. It is preferred to use containments with a volume for the medium of at least 100 000 l, more preferred at least 150 000 l and even more preferred at least 220 000 l. It is further preferred to use stirred tank reactors.

Additionally, it is preferred that the maximum filling volume ($V_R^{max.}$) of the containment for fermentation is at least 20 % higher the volume of the initial starting volume, wherein $V_R$ is the fermenter working volume.

The microorganisms of the present invention are cultured under controlled aeration. The term "controlled aeration" includes sufficient aeration (e.g., oxygen) to result in the production of the desired product. It is preferred that air is sparked through the fermentation containment, preferably the air is sparked at $\geq$ 1 vvm (volume of air per volume of medium per minute). The dissolved oxygen partial pressure ($pO_2$) can be measured during the fermentation process, e.g. with polarographic oxygen probes. The agitation speed and agitation can be used to control the $pO_2$, wherein a stepwise increase of the agitation speed might be necessary to control the $pO_2$ at preferable values $\geq$ 10%, more preferably $\geq$ 15%.

Additionally, it is preferred that the level of pantothenate production before the first withdrawal of medium is performed, is at a level of more than 25 g/l and further preferred more than 34 g/l.

Appropriate culture medium (or production medium) can be chosen by a person skilled in the art. An exemplary culture medium is given in the examples.

The feeding solution used for the semi-continuous fermentation process comprises glucose preferably in an amount of 500-800 g/l, further preferred 700-800 g/l. The feeding is performed at a continuous feeding rate, while a condition of glucose limitation is maintained ($q_{S/X(set)} \leq q_{S/X}^{max}$), wherein $q_{S/X}$ is the specific substrate uptake rate and the subscripts "S" and "X" represent the substrate (glucose) [g/l] and the biomass concentration [g/l], respectively. A suitable feeding range is from 4 g/l/h up to 41 g/l/h, further preferred 10 g/l/h up to 14 g/l/h. The introduction of the $\mu$-stat principle (control of the growth rate within a specific range and control of a constant growth rate) has been achieved using the equation:

$$F_S(t) = \frac{\mu}{Y_{X/S}S_F} \cdot X \cdot V \cdot (t_0) \cdot e^{\mu(t-t_0)},$$

wherein $\mu$ is defined as the bacterial growth rate, (1/h), $Y_{X/S}$ is the Biomass Yield (conversion efficiency of glucose into biomass), $S_F$ represents the substrate (glucose) concentration (g/l) in the feeding, X represents the biomass concentration (g/l), V represents the volume (l), F is the flow (g/h) and t represents the time. Further definitions used in the description: Y is the Yield (conversion efficiency), N is the N-source substrate [g/l], P is the Product concentration [g/l], $P_V$ is the Volumetric productivity [g/l/h], C is the Carbon source, N is the Nitrogen source ; Further subscripts or superscripts used in the description: W = Withdrawal, T = Transfer, H = Harvest.

The bacterial growth rate ($\mu$) can be determined by using the following equation (Biochemical engineering fundamentals, J. E. Bailey and D. F. Ollis; 2nd Edition, ISBN 0-07-Y66601-6; McGraw Hill International Editions, page 397):

$$\ln \frac{X_2}{X_1} = \mu \, (t_2 - t_1),$$

wherein $X_n$ ($X_1$, $X_2$) is the normalized-by-volume dry cell mass (determined by gravimetric methods known in the art, compare Example 6) at the timepoint ($t_n$). Furthermore, $t_n$ ($t_1$, $t_2$) is the timepoint of measuring the dry cell mass.

In order to control the level of $PO_4^{3-}$ during the fermentation process, a J solution (SCP-mix) is used. Exemplary formulations of the J solution are displayed in Table 2. Although shortage of any trace element might not occur during the fermentation process, it is additionally preferred that the same mixture and concentration as supplied in the production medium is included in the J solution.

The supplementation of the feeding solution with the J mixture (reformulation of feeding solution) was performed depending on the level of phosphate ions in the fermentation broth in order to avoid severe P-limitation, independently of the feeding intensity. The J addition is preferably performed to maintain a level of $PO_4^{3-} \geq 250$ mg/l, further preferred $PO_4^{3-} \geq 500$ mg/l and most preferred $PO_4^{3-} \geq 700$ mg/l in the fermentation broth. It is further preferred to make the first addition of the J solution after 48 hours.

The medium withdrawal is performed in a discontinuous manner. Preferably 10-20 %, further preferred 10-25 %, even further preferred 20-35 % by volume of the medium in the fermentation containment ($V_R$) at the time of the medium withdrawal is removed at certain time intervals, further preferred 10-20 % by volume. In addition, it is preferred that the medium withdrawal is at least performed two times during the fermentation process, further preferred four times, even more preferred at least 15 times.

The time intervals for the medium withdrawals can be chosen to achieve the highest product outcome. The term "interval" means the space of time between two medium withdrawals. It is preferred that the first medium withdrawal is performed not before 24 hours, further preferred not before 48 hours after starting the fermentation process. In addition, it is preferred that the following medium withdrawals are performed at the same time intervals. It is preferred to chose time intervals of less than 30 hours, further preferred of less than 25 hours and even more preferred of less than 20 hours and most preferred of 12 hours or less.

All the medium which has been withdrawn from the fermentation containment is collected in a buffer tank where a short pasteurization process is applied and then sent to the recovery line for the downstream operations.

Additionally the present invention includes a step of recovering the desired compound (e.g., pantothenate). The term "recovering" a desired compound includes extracting, harvesting, isolating or purifying the compound from the culture medium. In order to achieve high amounts of pantothenate, the culture medium of the main fermentation tank and the withdrawn medium is used.

Recovering the compound can be performed according to any conventional isolation or purification methodology known in the art including, but not limited to, treatment with a conventional resin (e.g., anion or cation exchange resin, non-ionic adsorption resin, etc.), treatment with a conventional adsorbent (e.g., activated charcoal, silicic acid, silica gel, cellulose, alumina, etc.), alteration of pH, solvent extraction (e.g., with a conventional solvent such as an alcohol, ethyl acetate, hexane and the like), dialysis, filtration, concentration, crystallization, recrystallization, pH adjustment, lyophilization and the like. For example, a compound can be recovered from culture medium by first removing the microorganisms from the culture. Medium are then passed through or over a cation exchange resin to remove cations and then through or over an anion exchange resin to remove inorganic anions and organic acids having stronger acidities than the compound of interest.

Preferably, a desired compound of the present invention is "extracted", "isolated" or "purified" such that the resulting preparation is substantially free of other medium components (e.g., free of medium components and/or fermentation byproducts). The language "substantially free of other medium components" includes preparations of the desired compound in which the compound is separated from medium components or fermentation byproducts of the culture from which it is produced. In one embodiment, the preparation has greater than about 80% (by dry weight) of the desired compound (e.g., less than about 20% of other medium components or fermentation byproducts), more preferably greater than about 90% of the desired compound (e.g., less than about 10% of other medium components or fermentation byproducts), still more preferably greater than about 95% of the desired compound (e.g., less than about 5% of other medium components or fermentation byproducts), and most preferably greater than about 98-99% desired compound (e.g., less than about 1-2% other medium components or fermentation byproducts). When the desired compound has been derivatized to a salt, the compound is preferably further free of chemical contaminants associated with the formation of the salt. When the desired compound has been derivatized to an alcohol, the compound is preferably further free of chemical contaminants associated with the formation of the alcohol.

In an alternative embodiment, the desired compound is not purified from the microorganism, for example, when the microorganism is biologically non-hazardous (e.g., safe). For example, the entire culture (or culture supernatant) can be used as a source of product (e.g., crude product). In one embodiment, the culture (or culture supernatant) is used without modification. In another embodiment, the culture (or culture supernatant) is concentrated. In yet another embodiment, the culture (or culture supernatant) is dried or lyophilized.

In order to determine the improved product concentration of the fermentation process according to the present invention, the pantothenate titer can be measured in the main reaction containment and in the containment containing the withdrawn medium. To determine the pantothenate titer can be performed using known methods in the art (WO 2005/103274 or Example 5). Preferably the overall product concentration is greater than 25 g/l, further preferred greater than 30 g/l, even further preferred greater than 35 g/l and most preferably greater than 45, 60, 80 g/l.

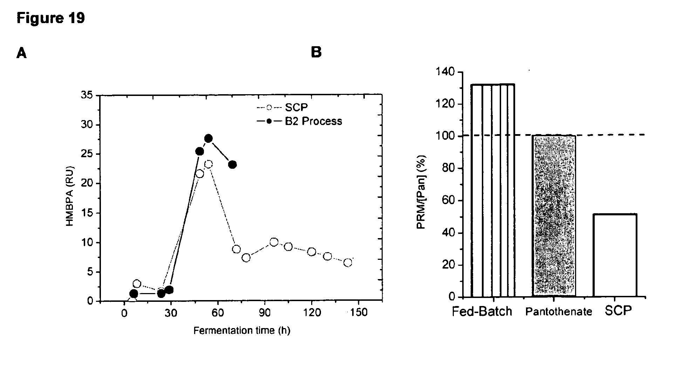

Furthermore the present invention can be used to decrease the amount of pantothenate-related-metabolites (PRM). For example the amount of HMBPA can be decreased by at least 40 %, preferably by 60 % even further preferred by 75% compared to the standard fed-batch process. HMBPA was reduced from 23 g/l down to -7 g/l when the process was changed from fed-batch to semi-continuous (Fig. 19).

**Description of the Figures:**

**Figure 1a and 1b** display the metabolic pathway of the pantothenate synthesis and related metabolites

**Figure 2** shows the growth and production kinetics using $B_2$ recipe in a 20l reactor with 6l initial volume

**Figure 3** shows the kinetics relationships of pantothenate production using $B_2$ recipe. $Y_{P/S}$ and $q_{P/X}$ are plotted against $\mu$. $Y_{P/S}$ is a derivative yield taking every 4h using the fitting plots of experimental data, wherein $Y_{P/S}$ is defined as the Product Yield (conversion efficiency of glucose into product) and $q_{P/X}$ defined as the specific productivity (g product/g biomass/h)

Figure 4 (Example 10) represents a schematic representation of "in vivo" pathway assessment undertaken to evaluate product toxicity and capability of *Bacillus* cells to overproduce pantothenate.

**Figure 5** (Example 10) displays the III phases assessment of the pantothenate pathway of *B. subtilis* using fed-batch operation mode. Phase I, batch phase, cells are grown in glucose excess. Phase II, glucose + pantoate feeding was applied. Phase III, glucose + pantoate + β-alanine were supplied.

**Figure 6** (Example 10) shows the intensification of conditions applied in Phase III of Figure 5. All three substrates were fed right form the beginning of the fed-batch phase. Fermentation was stopped at 104 h due to shortage of pantoate availability

**Figure 7A and B** (Example 11) display the time course profiles of $NH_4$, $PO_4$ and OD (optical density as indicator for the biomass evolution) in an extended pantothenate fed-batch fermentation using $B_2$-recipe.

**Figure 8** (CalPan process from Example 12) displays pantothenate titers at the end of the fermentation time (51 h $\pm$ 3) carried out with different feeding profiles. The runs labeled as "1" in Figure 8 are runs conducted up to 65h, while the runs labeled as "2" were up to 70h.

**Figure 9** (CalPan process from Example 12) shows the Final Volumetric productivities achieved with different feeding profiles. The runs labeled as "1" and "2" are the same as in Figure 8.

**Figure 10** (CalPan process from Example 12) displays the response of the final cumulative yield to the variable feeding profiles and medium adjustments applied. The runs labeled as "1" and "2" are the same as in Figure 8.

**Figure 11** (CalPan process from Example 12) shows the process intensification for pantothenate production by increasing feeding profile and supplementing medium composition compared to the reference process ($B_2$ fermentation process).

**Figure 12** (semi-continuous process from Example 13) displays the screening by expression level. The selection criterion used was gene expression > 10000, thus all genes having lower expression than this setting point were filtered out. Total genes population = 4,088.

**Figure 13** (semi-continuous process from Example 13) displays the introduction of withdrawals to manage the working volume $\leq V_R^{max}$ (maximum filling degree) and extended fermentation operated on semi-continuous mode.

**Figure 14a and b**. (Example 14) time course profiles of pantothenate titer (A) and overall product output (B) when applying SCP for over 140h fermentation process

**Figure 15a and b**. (Example 14) Yield (A) and Productivity (B) profiles of SCP as compared to the reference process

**Figure 16**. (Example 15) Time course profiles of pantothenate concentration (A) and cumulative output (B) utilizing SCP design as compared to the reference process.

**Figure 17**. (Example 15) Conversion efficiency (A) and volumetric productivity (B) as a function of the process design applied

**Figure 18.** (Example 15) Gene Expression in CalPan semi-continuous process (SCP) using *B. subtilis* ($P_{15}panBCD$ $P_{15}$ $panE$).

**Figure 19**. (Example 15) Pantothenate Related Metabolites (PRM) as function of the fermentation operation mode. **(A)** Time course profile of HMBPA, **(B)** Percentage change when normalized to the pantothenate level. PRM is the lumped sum of HMBPA (3-(2-hydroxy-3-methyl-butyrylamino)-propionic acid), DHMBPA (3-2,3-dihydroxy-3-methyl-butyrylamino)-propionic acid, HMBSA (3-(2-hydroxy-3-methyl-butyrylamino)-succinic acid) and 2,3 Dyhydroxybenzoate (Figure 1b).

**Example 1: Strain**

*Bacillus subtilis* overproducer of pantothenate was used to develop the process according to the present invention. The strain selected for all examples was *Bacillus subtilis* having the genotype: ***P₁₅panBCD P₁₅panE.*** This strain is derived from a strain which has the operon *P₁₅panBCD* engineered. This was accomplished by first isolating promoter deletion mutants, and then using these auxotrophic strains to insert strong, constitutive promoters in front of the biosynthetic operon/gene by selecting for restoration of prototrophy. The same procedure was applied to engineer *panE* (WO 01/21772).

**Example 2: Culture Medium**

The composition of **VYS** medium is as follows (g/l): **V**eal infusion broth, 30; **Y**east extract, 5; Sorbitol, 10; $K_2HPO_4$ 2.5. VYS medium was used in the first stage of the inoculum.
In the second stage inoculum and in the main culture the following medium formulation was applied (g/l): glucose, 27.3; Yeast extract, 12; Na-glutamate, 1; $KH_2PO_4$, 6.28; $K_2HPO_4$, 6.28; $Na_2HPO_4 \cdot 2H_2O$, 11; $NH_4Cl$, 0.31; $(NH_4)_2SO_4$, 1.88, Antifoam Basildon, 0.2; $MnSO_4 \cdot H_2O$, 0.017; $CoCl_2 \cdot 6 H_2O$, 0.005; $(NH_4)_6Mo_7O_{24} \cdot 4H_2O$, 0.0018; $AlCl_3 \cdot 6 H_2O$, 0.0012; $CuCl_2 \cdot 2H_2O$, 0.0009; $MgSO_4 \cdot 7H_2O$, 1.2; $ZnSO_4 \cdot 7H_2O$, 0.005; $CaCl_2 \cdot 2 H_2O$, 0.0625; $FeSO_4 \cdot 7H_2O$, 0.05. Glucose and the trace elements were sterilized separately.
The basal feeding solution contains (g/l): Glucose, -750-800; $MgSO_4 \cdot 7 H_2O$, 2; $MnSO_4 \cdot H_2O$, 0.015; $ZnSO_4 \cdot 7 H_2O$, 0.004.

**Example 3: Inoculation Procedure**

Inoculum preparation was carried out in two stages. In the first stage 1 ml stock culture, previously prepared and preserved at -25°C, was inoculated into 25 ml VYS broth supplemented with tetracycline (15 $\mu$g/ml) in a 100 ml Erlenmeyer flask, which was then incubated for 7 h at 39°C on a rotary shaker at 200 rpm. In the second stage, 3 ml of this culture (1 % v/v) was transferred to 300 ml of the production medium containing 15$\mu$g/ml chloramphenicol. This second pre-culture was carried out in a 2l Erlenmeyer flask and was incubated again at 39°C for 16 $\pm$ 1h, time at which an OD > 14 was achieved. For the final stage, the content of such flask was transferred aseptically to the stirrer tank reactor (fermenter) to give approximately 5% w/w inoculum concentration.

**Example 4: Reactor and Operating Conditions**

Fermentations were carried out in a 20l New Brunswick (NB) fermenters (USA) for most of the studies reported here (Examples 9-12), while 15l stirred tank reactors Fa. BBraun (Germany) were employed for the Semi-Continuous Process (SCP). The initial working volume was set-up at ~6 and ~5l in NB and BBraun, respectively. The maximum volume depended upon the experiment in question. The temperature was controlled at 39°C, and the pH was kept at 6.8 $\pm$ 0.1 by controlled addition of $NH_4OH$ 27%. Air was sparged at $\geq$ 1 vvm, as required. The dissolved oxygen partial pressure ($pO_2$) was measured with polarographic oxygen probes. A back pressure of 500 mBar was applied. The initial agitation speed was set at 400 rpm with stepwise increase as required by the $pO_2$ control.

The $pO_2$ was controlled at values $\geq$ 15% by a combination of aeration and agitation using a PID controller action. This controller has one inner loop (a slave) involving the measurement of agitation speed and an outer loop (the master) involving the $pO_2$ measurement. The output of the $pO_2$ loop controller serves as the set point for the agitation loop. Once the agitation speed reached the upper limit (1000 $\pm$ 50 rpm), the control is switched to the aeration loop. The aeration flow was adjusted in a stepwise form from 6 NL/min (throughput) to 12 NL/min as the lower limit of $pO_2$ was approached.

**Example 5: Determination Pantothenate and Pantothenate-Related Metabolites**

Chromatography of samples was performed on a Phenomenex LUNA C8 column, using an Agilent 1100 HPLC system equipped with a thermostatted autosampler and a diode array detector. The column dimensions are 150 x 4.6 mm, particle size 5 micron. The column temperature was kept constant at 20°C. The mobile phase is a mixture of 0.1% acetic acid (A) and methanol (B). Gradient elution is applied, ranging from 1% B to 45% B in 15 minutes. The flow rate is 1 ml/min. Pantothenate was monitored using UV absorption at 220 nm, and is eluted at approximately 9.6 min. The calibration range of the method is from 1 to 100 mg/l pantothenate. Other pantothenate related metabolites are also separated and measured by this method.

**Example 6: Cell Dry Weight**

Cell dry weight concentration and the dry cell mass (normalized-by-volume) was determined in duplicate by gravimetric methods. The culture broth was diluted as required and then centrifuged in 2 mL Eppendorf tubes at 14000 rpm at 4°C for 10 min to settle cells. The supernatant was decanted and saved for other analytical determinations. The cell pellet was washed and then centrifuged again under the same conditions described for the sample centrifugation. After this second centrifugation the washing water was discarded and the cell pellet was dried under vacuum at 40°C and weighed.

**Example 7: Determination of Ammonium**

The determination of ammonium ions was done with an automatic analyser 'Vitros DT II' that works with special coated reaction slides. The slides are multilayered and contain immobilized/coated analytical elements on a plastic support. 10 (L of sample containing ammonium in the range of 1-500 (Mol was deposited on the slide and evenly distributed by the spreading layer. The ammonia in the sample then diffuses through a semi-permeable membrane into a layer containing bromphenol blue as the indicator dye. By measuring the amount of light reflected from the dyed layer after a fixed incubation period, the analyzer calculates the amount of ammonia present in the sample.

**Example 8: Determination of Phosphate**

The determination of inorganic phosphorus ions was done with the automatic analyser Vitros DT II system. This system works with special coated reaction slides that contain all the reagents necessary to determine phosphorus levels in 10 $\mu$L sample.

10 $\mu$L fermentation supernatant containing PO43- ions in the range of 0.81-1.45 mmol/l is deposited on the slide and evenly distributed by the spreading layer. The ion determination is based on the reaction of phosphorus with ammonium molybdate to form a complex that, after chemical reduction, has a strong blue color. The intensity of the color is proportional to the amount of phosphorus in the sample. The reaction sequence is provided below:

$$PO_4^{-3} + NH_4 \text{ molybdate} \xrightarrow{pH = 4.2} NH_4\text{-phospho-molybdate} \qquad \text{Equation (1)}$$

$$NH_4\text{-}phospho\text{-}molybdate \xrightarrow{\ p\text{-}Methylamin\ ophenolsulphate\ } Heteropoly\ molybdate\text{-}blue \qquad \text{Equation (2)}$$

**Example 9: Evaluation of B2-process for Pantothenate Production**

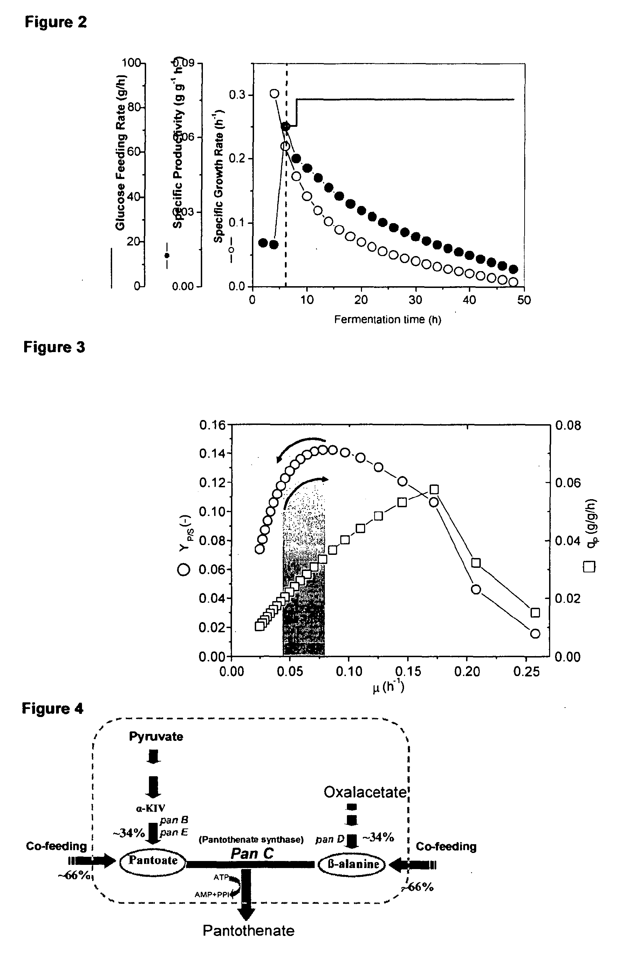

The *Bacillus subtilis* strain ($P_{15}$ *panBCD* $P_{15}$ *panE*) was used for the production of pantothenate (Vitamin $B_5$). In a first step, the standard fed-batch process of $B_2$ was adopted (US 2005/01 240 30). This fed-batch process is carried out using complex medium formulation. It comprises a short initial batch phase of approximately 6h followed by C-limited fed-batch operation of around 42h. Consequently, during the initial batch stage of cultivation the growth rates are determined by the complex N-source in use (yeast extract), while the glucose feeding pattern overtakes such control during the fed-batch mode. The feeding pattern is somehow oversimplified, as the C-source supplied in the batch phase is depleted and $pO_2$ increases drastically, glucose feeding starts at the rate of ~13.3 g/l/h for 2 h and then increases to a moderate 14.7 g/l/h and remains constant towards the end of the fermentation. Figure 2 shows the feeding rate for lab scale and the kinetics associated to it. Moreover, conditions of N-excess are maintained by the pH control, which uses $NH_4OH$ at lab scale and ammonia gas at full production. Figure 2 shows the growth and production kinetics using $B_2$ recipe in a 201 reactor with 61 initial volume.

Using this process recipe and the operating conditions as well as medium formulation described in Example 2, a set of fermentation runs were conducted to produce pantothenate by *B. subtilis.* Table 1 summarizes the outcome of six runs. The performance indexes shown in Table 1 are highly reproducible as shown by the low standard deviations. At first it could be concluded that a $B_2$ recipe could well be used for $B_5$ production using the same microorganism *B. subtilis* (genotype $P_{15}panBCD$ $P_{15}panE$). It is within this context that the kinetics characteristics of $B_2$ recipe were evaluated in more detail. As seen in Figure 2, both $\mu$ and $q_{P/X}$ reduce gradually towards the end of the fermentation. Moreover, the similar decay profile seems to insinuate that a direct relationship may exist between $\mu$ and $q_{P/X}$. To further evaluate this, $q_{P/X}$ was plotted against $\mu$ in Figure 3.

Additionally, the conversion efficiency of glucose into pantothenate (differential $Y_{P/S}$) was evaluated as a function of growth ($\mu$) in the same figure. Figure 3 shows clearly that an optimum non-coincidental $\mu$-range exits for either $Y_{P/S}$ or $q_{P/X}$ and hence the optimization criteria will very much depend upon the economic targets of choice. For instance it could well be that $q_{P/X}$ will have to be compromised in favor of high $Y_{P/S}$ as shown by the shadowed window in Fig. 3. It becomes also clear that extremely low values of $\mu$ should be avoided if a cost-efficient process is what matters. In summary, the results of Fig. 3 provides design alternatives for process intensification aiming at enhanced fermenter throughput.

**Table 1.** Fermentation performance indexes of *B. subtilis* (genotype $P_{15}panBCD$ $P_{15}PanE$) using fed-batch process as established for B2 production. $Y_{P/S}$ is the cumulative yield after 48h of fermentation time

| Run Code | [P] (g/l) | $Y_{P/S}$ (-) | $P_V$ (g/l/h) | Pan total (g) |
|---|---|---|---|---|
| *CBioF011* | 27.80 | 0.098 | 0.58 | NA* |
| *CFBat018* | 27.33 | 0.111 | 0.57 | 260.7 |
| *CFBat026* | 24.19 | 0.090 | 0.51 | 256.5 |
| *CFBat028* | 27.33 | 0.111 | 0.57 | 260.7 |
| *CFBat035* | 26.10 | 0.104 | 0.54 | 240.2 |
| *CFBat040* | 26.80 | 0.107 | 0.56 | 237.5 |
| * Only run conducted in 3 L fermenter | | | | |
| **Average** | **26.59** | **0.10** | **0.55** | **251.12** |
| STD Dev. | 1.31 | 0.008 | 0.03 | 11.37 |

**Example 10: Limits of Pantothenate Biosynthesis**

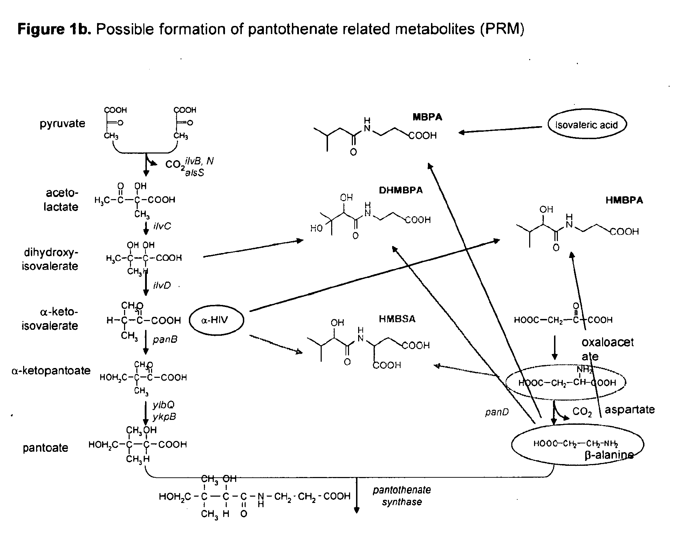

The previous example strongly suggests that the standard fed-batch process as applied for $B_2$ production may be sub-optimal for pantothenate production and that enough room for process intensification exists. Before any effort of process intensification was undertaken it was felt that two critical questions remained to be answered. Firstly, are high levels of pantothenate toxic to the cells? Secondly, if toxicity is not at play, what would be the highest production capacity of the strain assuming that all biosynthetic genes are over-expressed and hence no limitation in the building-blocks pantoate and β-alanine exists? To address both questions using a single experimental setting a co-feeding experiment was devised. While keeping the pantothenate pathway active (Figure 1a/ 1b) in a fed-batch cultivation of *Bacillus subtilis* (genotype $P_{15}panBCD\ P_{15}panE$), excess of β-alanine and/or pantoate pools were generated by external co-feeding of these precursors (Figure 4). This way, both branching pathways leading to pantoate and β-alanine, as well as the condensation step by pantothenate synthase (*panC*) were assessed independently. To carry out this experiment, pantoate, which is not commercially available, was produced in limited amounts.

Addition of approximately 8 g/liter β-alanine to the fermentation during glucose feeding resulted in only a marginal increase in pantothenate production, which was around 8%. This increase is significantly less than the theoretical titer increase (~80%) expected if all the β-alanine was converted into pantothenate. As judged by the results most of the β-alanine was channeled into biomass and PRM (namely HMBPA) suggesting that enhancement of one single branching pathway does not result in increased pantothenate. This preliminary observation was further supported by the results described below.

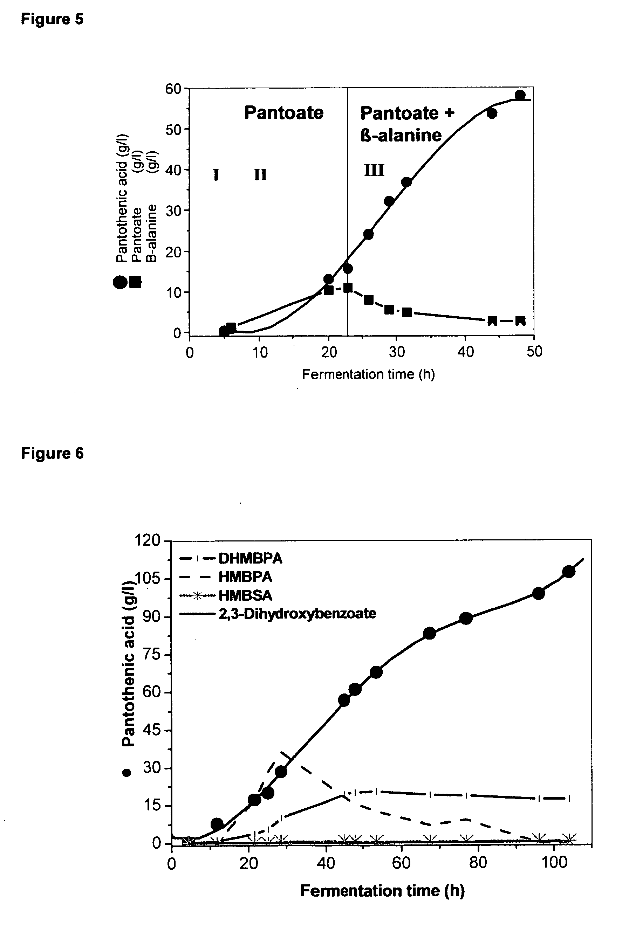

Although pantoate co-feeding started at very low rate (7% of the glucose feeding), within the first 23h of cultivation pantoate accumulated up to 10 g/l (Fig. 5). Furthermore, pantothenate synthesis was not improved as expected suggesting limitation at the complementary branching pathway. After addition of β-alanine the accumulated pantoate was depleted within few hours stimulating the pantothenate synthesis. Moreover pantoate co-feeding could be increased gradually from 7% up to 28% with the concomitant increase of pantothenate synthesis reaching 58 g/l within 48h as shown in Figure 5.

Clearly, the enhancement of pantothenate synthesis requires the optimization of both branching pathways simultaneously. Based on the above findings pantothenate synthesis was further boosted by extending the cultivation time, so as to be able to achieve higher product concentration and assess the potential of pantothenate synthase (*pan C*). By balancing both co-feedings (pantoate and β-alanine), over 107 g/l of pantothenate was produced within 104 h (Figure 6). So far such level of pantothenate was not reported in the literature. At 104 h cells were highly active, as can be judged by the upward trend of pantothenate accumulation. The fermentation run was stopped due to shortage in pantoate supply rather than saturation of reaction. These results clearly demonstrate the ability of *B. subtilis* to produce high levels of pantothenate without adverse effect on cell growth or viability. Toxicity or inhibitory effect should be then ruled out for cultivations aimed at titers below 107 g/l.

### Example 11 Medium Assessment and Enhancement of its Nutritional Power

The two previous examples showed that process intensification is possible without being concern about product toxicity, a condition that is strive for in the biotech production of 'low cost large volume' products. Process intensification could well be managed by increasing the feeding rate and changing its pattern along the fermentation time. Higher turnover of medium supply may however cause dilution and even depletion of the key nutrient factors that are added to the initial working volume. Therefore a quick look into the medium supply was warranted.

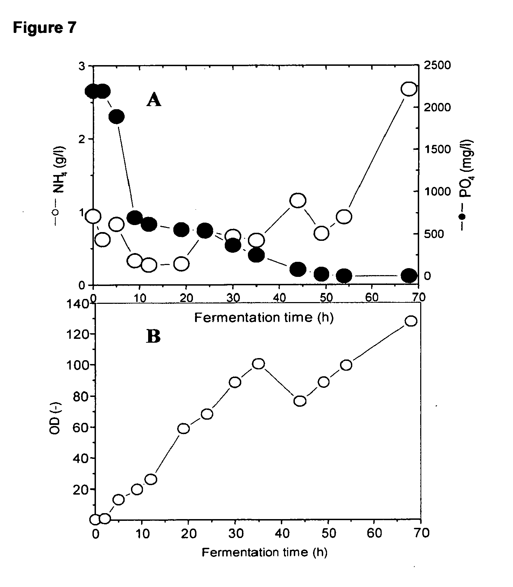

Without changing the feeding profile, the 'dilution effect' was rather tested by extending the fermentation time from 48h up to 68h. All the operating conditions were kept the same as established in the $B_2$ process. A close follow-up of ammonia and phosphate ions showed interesting insights (Figure 7A). While $NH_4$ showed an upward trend, the phosphate ions were depleted as early as 44 h of the cultivation time, indicating P-limiting conditions. More interesting is the profile of cell density as judged by the OD profile in Fig. 7B. The OD reached a peak number of 100 at -35h but then went down to ~76 within the following ~9 h. This coincided with the time (44h) at which conditions of P-limitation were encountered. Surprisingly, after this decay cell density recovered slowly and showed an upward trend again. Such a profile seems to indicate cell lysis, which is well known in *B. subtilis* cultivations under stress conditions. Cannibalism by sporulating *B. subtilis* was reported by Gonzáles-Pastor et al. (González-Pastor et al., Science 2003, 301: 510-513.). These authors reported that triggered by nutrient limitation cells enter the sporulation pathway releasing a killing factor and a signaling protein that act cooperatively to block sister cells from sporulating and to cause them to lyse. Sporulating cells feed on the nutrients thereby released, which allows them to keep growing rather than to complete morphogenesis.

Based on the above observations a readjustment of the feeding medium was found appropriate. To this end the feeding solution was reformulated (J, SCP-mix solution was formulated) (Table 2). Furthermore, although shortage of any trace element could not be determined due to equipment limitation, the same mixture and concentration as supplied in the production medium was included in the J solution. The supplementation of the feeding solution with the J mixture was devised to be dependent on the level of phosphate ions in the fermentation broth, this way severe P-limitation can be avoided, independently of the feeding intensity. To facilitate operation a criterion for J addition was set up by defining the lower boundary and establishing a set-point of $PO_4^{3-} \geq 500$ mg/l.

**Table 2.** Formulation of J solution for process intensification

| J Solution Composition | g/l Feeding Solution |
| --- | --- |
| $KH_2PO_4$ | 8.10 |

(continued)

| **J** Solution Composition | g/l Feeding Solution |
|---|---|
| $K_2HPO_4$ | 8.10 |
| $Na_2HPO_4 \cdot 12H_2O$ | 14.76 |
| $NH_4Cl$ | 0.45 |
| $(NH_4)_2SO_4$ | 2.52 |
| Trace elements | Same as in Example 2 |

**Example 12: Process Intensification for Fed-batch Fermentation: Development of CalPan Process (CP)**

The development of an alternative distinct CalPan process, was based upon increased and variable feeding profile, as well as medium reformulation. The former in order to modify *B. subtilis* growth and enhance glucose uptake rate $q_{S/X}$ ($q_{S/X}$ is defined as the specific substrate uptake rate (g substrate/g biomass/h)).
The later, so as to be able to prevent limitation of key-nutrients (non glucose) due to the higher turnover of biomass and dilution effect coming from the higher feeding rates. This was accomplished by:

- While conditions of glucose limitation was maintained ($q_{S/X(set)} \leq q_{S/X}^{max}$) and the dissolved oxygen level ($pO_2$) was kept $\geq$ 15%, different feeding profiles ranging from 4 g/l/h up to 41 g/l/h were applied in fermentations using 6 l as initial working volume $V_R$ ($t_0$). This also included the introduction of the $\mu$-stat principle after the equation below:

$$F_S(t) = \frac{\mu}{Y_{X/S}S_F} \cdot X \cdot V \cdot (t_0) \cdot e^{\mu(t-t_0)} \qquad (3)$$

- The basal medium as described in Example 2 was increased by a factor of 1.3 in order to increase the biomass density at the at the end of the batch phase and be able to apply higher feeding rates.

- J solution was introduced as described in Example 11

- Filling degree of the reactor $V_R(t_f) \leq$ 15 l ($t_f$ = final time, 48 to more than 100 h), which is 75% of the full volume (20 l New Brunswick Stirred Tank Reactors)

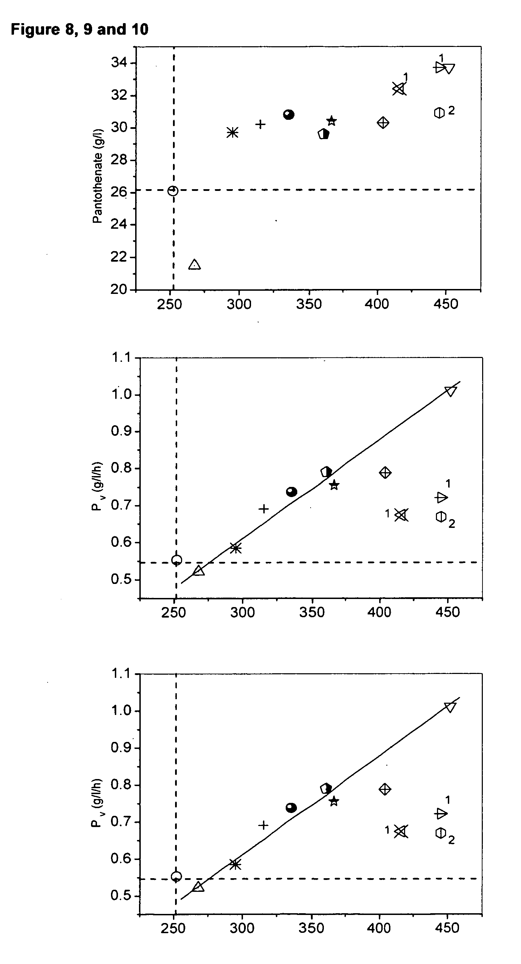

A total of 12 fed-batch fermentations were conducted using these process settings. Figures 8 to 10 show the 'end of fermentation' results by depicting each of the performance indexes against the total product output. The fermentation time was in average 51h $\pm$ 3h, except for the three runs labeled with 1 or 2, which indicate longer fermentations; 65 and 70h, respectively. From these results interesting relationships could be observed. As seen in Fig. 8, pantothenate titer could be enhanced from 26 g/l up to ~ 34 g/l. While high product output could be inferred as a result of higher titer, this was not absolutely right for the runs having lower titer but comparable output. Similarly, at the intermediate value of 30 g/l pantothenate the final output ranged from around 300 to about 450 g (Figure 8). This implies that the feeding rate had the major impact by changing the filling degree of the fermenter and hence changing the harvesting volume. In fact $V_R(t_f)$ ranged from 9.2 l in the reference run ($B_2$-like) up to 14.4 l in the CPs trials. An additional factor affecting output is the fermentation time, as seen for those runs extended up to 65-70 h, which show higher output without necessarily achieving the highest titer.

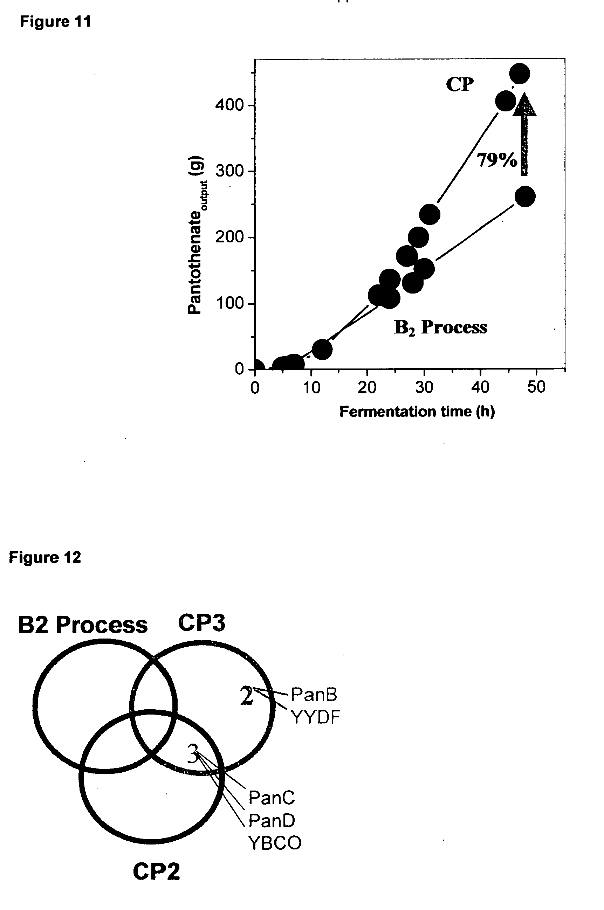

While the product output could successfully be enhanced by almost 80%, a key economic factor is the fermentation time required to achieve that step change, thus the productivity. In general, a linear relationship between output and productivity could be inferred from the data shown in Figure 9, except again for those runs having longer fermentation time and hence slower feeding profile.

While high product output with efficient time utilization and above that with high titer could be achieved (see arrows in Figs. 8 and 9), another crucial question that still remained to be answered was how much glucose we need to invest to achieve that. Interestingly, all the final cumulative yields lined up at the same level as the reference run ($B_2$-process). The final integrated pantothenate yield ($Y_{P/S}$) was almost independent of the feeding profile, it reminded around 0.10 $\pm$ 7%. Encouraging was that the final $Y_{P/S}$ was not affected negatively by the increase in the feeding rate, which otherwise could have had negative economic consequences. Process intensification at expenses of higher raw materials cost is less desirable.

The performance indexes shown in Figures 8 to 10 allowed to screen those process variables that best matches the optimization criteria of high fermenter throughput with minimum operation cost. The best run identified (marked with arrows in Figs. 8 to 10) corresponded to the 48h fed-batch having a feeding profile ranging from 62 to 86 g/h. This run showed enhancement of the pantothenate output by almost 80% without diminishing product yield. The time course profiles as compared to the base line process are provided in Figure 11.

Furthermore, the above screening process was supported by the use of DNA microarrays in order to gain insights into the genome-wide response to changing cultivation conditions as applied during the process intensification. To this end, the custom-made *B. subtilis* RoRBS01a GeneChip was employed (Lee et al., J. Bacteriol 2001, 183, 7371-7380). GeneChips experiments were performed for the $B_2$-reference process as well as for two CP processes. Unbiased global assessment of about 4,088 gene expression showed that operating conditions as applied in $CP_2$ and $CP_3$ had the ability of maximizing the pan genes as compared to the $B_2$-process design (Fig. 12).

As mentioned above, the other pillar of the process intensification was the medium supplementation. Therefore to complete the evaluation of this example, a more thorough examination of the macro & micro nutrients at the end of the fermentation was felt necessary. To accomplish it, final fermentation samples of two CP processes having on one hand moderate constant feeding profile (as in the $B_2$-process) and the other hand, variable high feeding rates were analyzed. The results are depicted in Table 3 together with the initial ($t_0$) supplied dosage.

**Table 3.** Macro and micronutrients profiling at the end of the fermentation as compared to the initial dosage provided at the beginning of the process (Basal medium)

| Macro & Micro Elements | Basal Medium | CP with $B_2$ Feeding | CP Feeding |
|---|---|---|---|
| | mg/kg | mg/kg | mglkg |
| | | | |
| cadmium | | 0.05 | 0.04 |
| calcium | 17.04 | 2.25 | 4.91 |
| cobalt | 1.24 | 0.97 | 0.77 |
| phosphorous | 2621.52 | 2020.00 | 1510.00 |
| potassium | 2410.84 | 3410.00 | 3100.00 |
| copper | 0.34 | 0.20 | 0.20 |
| lead | | 1.00 | 1.00 |
| magnesium | 118.40 | 51.50 | 64.80 |
| manganese | 5.53 | 0.33 | 0.30 |
| sodium | 620.70 | 1310.00 | 1230.00 |
| nickel | | 0.30 | 0.30 |
| iron | 10.05 | 2.82 | 2.28 |
| zinc | 1.14 | 1.01 | 0.53 |
| sulfur | 508.32 | 413.00 | 348.00 |

The formulation and dosages in the basal medium ($t_0$, starting point of fermentation) were calculated using the nutrients matrix developed for CalPan (Table 4) and the medium composition described in Example 2. As shown by the results in Table 3, no severe limitation of the key nutrients could be found. However attention should have to be paid to Mn if further and more drastic intensification conditions are to be taken. The similar and slightly higher levels of P, K, and Na in CP runs as compared to the basal medium could be explained by the extra contribution of the J solution (Example 11). Cd, Pb, and Ni are contribution of the Yeast extract alone. Nevertheless these levels are lower than the limit of ≤ 10 mg/kg established by the FCC (Food Chemical Codex). It can be derived that there are no dramatic differences in the elements profiling using different feeding profiles. This is because all feeding solutions had the same final composition independent of the feeding rate. Thus, if faster reactions were generated by the faster feeding, higher amount of a given compound per volume of reaction was concomitantly supplied.

EP 1 918 383 A1

**Example 13: Development and Validation of Semi-Continuous Process (SCP) for CalPan production.**

The results in Example 12 showed that fermentation throughput can directly be influenced by the feeding rate and the operation time. An extended fermentation while maintaining high reaction rates ($P_V$) and keeping the optimum conversion efficiency ($Y_{P/S}$) can be the best targeted conditions to achieve high fermenter output, a fact that is generally strived for in an attempt to achieve an effective production of "low cost -high volume" biotech products. Therefore efforts

**Table 4.** Matrix Approach for the calculation of Macro and Micro nutrients supply

| Components | MW | Ca | Co | P | K | Cu | Pb | Mg | Mn | Na | Fe | Zn | S |
|---|---|---|---|---|---|---|---|---|---|---|---|---|---|
| NH4OH | 35 | | | | | | | | | | | | |
| NH4NO3 | 80 | | | | | | | | | | | | |
| NH4Cl | 53.45 | | | | | | | | | | | | |
| (NH4)2HPO4 | 132 | | | | | | | | | | | | |
| Na-Glutamat | 187.14 | | | | | | | | | 12.285 | | | |
| KH2PO4 | 136.07 | | | 22.76 | 28.736 | | | | | | | | |
| K2HPO4 | 174.17 | | | 17.78 | 22.45 | | | | | | | | |
| Na2HPO4 * 12 H2O | 358 | | | 8.651 | | | | | | 6.4218 | | | |
| (NH4)2SO4 | 132 | | | | | | | | | | | | 24.3 |
| FeSO4.7H2O | 277.85 | | | | | | | | | | 20.101 | | 11.54 |
| NaCl | 58.45 | | | | | | | | | | | | |
| Fe Cl3 | 162.2 | | | | | | | | | | 34.433 | | |
| MnSO4.H2O | 169.01 | | | | | | | | 32.507 | | | | 18.98 |
| CoCl2.6H2O | 237.83 | | 24.778 | | | | | | | | | | |
| CuCl2x2H2O | 170.45 | | | | | 37.28366 | | | | | | | |
| MgSO4.7H2O | 246.38 | | | | | | | 9.8669 | | | | | 13.02 |
| ZnSO4.7H2O | 287.46 | | | | | | | | | | | 22.75 | 11.16 |
| CaCl2x2H2O | 146.98 | 27.269 | | | | | | | | | | | |

| Media | g/l | Ca | Co | P | K | Cu | Pb | Mg | Mn | Na | Fe | Zn | S |
|---|---|---|---|---|---|---|---|---|---|---|---|---|---|
| Yeast Extract | 12 | | | | | | | | | | | | |
| Na-Glutamat | 0.75 | | | | | | | | | 0.0921 | | | |
| | | | | | | | | | | | | | |
| KH2PO4 | 4.71 | | | 1.072 | 1.3534 | | | | | | | | |
| K2HPO4 | 4.71 | | | 0.838 | 1.0574 | | | | | | | | |
| Na2HPO4 * 12 H2O | 8.23 | | | 0.712 | | | | | | 0.5285 | | | |
| NH4Cl | 0.23 | | | | | | | | | | | | |
| (NH4)2SO4 | 1.41 | | | | | | | | | | | | 0.343 |
| FeSO4.7H2O | 0.05 | | | | | | | | | | 0.010 | | 0.006 |
| MnSO4.H2O | 0.017 | | | | | | | | 0.0055 | | | | 0.003 |
| CoCl2.6H2O | 0.005 | | 0.0012 | | | | | | | | | | |
| CuCl2x2H2O | 0.0009 | | | | | 3.4E-04 | | | | | | | |
| MgSO4.7H2O | 1.2 | | | | | | | 0.1184 | | | | | 0.156 |
| ZnSO4.7H2O | 0.005 | | | | | | | | | | | 0.001 | 6E-04 |
| CaCl2x2H2O | 0.0625 | 0.017 | | | | | | | | | | | |
| **Total** (g/L) | | 0.017 | 0.0012 | 2.622 | 2.4108 | 0.000336 | | 0.1184 | 0.0055 | 0.6207 | 0.0101 | 0.001 | 0.508 |
| **Total** (mg/L) | | 17.043 | 1.2389 | 2622 | 2410.8 | 0.335553 | | 118.4 | 5.5262 | 620.65 | 10.05 | 1.137 | 508.3 |

14

were focused in extending the fermentation process beyond 100 h. To do so a medium withdrawal mechanism was introduced, so as to be able to match with the extra reactor capacity demanded by the continuous feeding over a longer period of time. Such medium removal was planned to be executed on intermittent basis in a volume range of for example 10 %-20 % of the total working volume in the fermenter at the time of the medium withdrawal (Figure 13).

This Example shows two experimental validations of the use of Semi-Continuous Process (SCP) for the production of pantothenate by *B. subtilis.* The selection of process variables and operating conditions of SCP were built up on the results and observations of the preceding Examples, but this time using 15 l BBraun reactors. The following settings were applied:

- Initial working volume set to $V_R$ ($t_0$) ≈ 5 l (Lower than Example 12 due to the lower reactor size).
- Operation time > 100 h.
- Continuous feeding at rates within the optimum range identified in Example 12 but adapted to new reactor size.
- Employ same nutrients supplementation (J solution) and basal medium formulation, as described in Examples 11 and 12.
- Set up maximum filling degree as $V^{max}_R$ ≈ 10 l (including air and agitation) to allow visual control of the filling limit. The glass window of the fermenter reaches just up to 10 l.
- Withdrawals were designed to be managed as follows: around 10%-20% of the working volume was removed as fast as possible when the $V^{max}_R$-set was reached. This operation was repeated as many times as necessary (Fig. 13).
- Overall process design aimed at achieving pantothenate titers > 34 g/l at the time at which the first withdrawal had to be executed (-48h).

### Example 14: Validation of SCP Operation Mode Using Lab Raw Material

With the application of the variables above indicated, for the first time the cultivation of B. *subtilis* could be extended from the standard 48 h up to 143 h (Fig. 14) without impairing the conversion efficiency. Product concentration kept upwards trend reaching titers up to 45 g/l, which is significantly higher than the 48h reference process. Concomitantly, the cumulative pantothenate output improved steadily over the 143h with a slope of ~6 g/h (Fig. 14B). In total, 19.24 l (Equation 4) of fermentation broth was harvested having pantothenate titers in the range of 34-45 g/l. On operational terms, this volume represents more than 2 times the harvesting volume when using the standard fed-batch process as applied in $B_2$.

$$V_H = V_R(t_f) + \int_{t0}^{tf} V_w(t), \qquad \text{Equation(4)}$$

wherein $t_f$ means final time.

An analysis of the cummulative yield profiles (Figure 15A) shows a clear improvement in the first 48 h, but then levels off at similar levels as the reference process and remains unchanged until the end of the process. Such profile is certainly the result of the feeding profile affecting bacterial growth and hence production as observed in Example 8 (Fig. 3). Cell density reached titers up to -90 g/l and remained at that level showing a pattern similar to that of pantothenate concentration (Fig. 14A)

The volumetric productivity on the other hand (Fig. 15B), reached a maximum level at 48h and remained steady towards the end of the fermentation, which was expected when looking at the slope of Fig. 14B. Overall, when translating the results of Figure 14B into operational meaning, the fermenter throughput was improved by more than 2 times. If -855 g of pantothenate could be produced by using SCP in 143 h (Fig. 14 B), ~402 g would have to be harvested in the same time period using standard fed-batch process and the same fermenter size. The calculation of 402 g refers to two consecutive fed-batch runs plus the required turn around time between these runs.

### Example 15: Validation of SCP Fermentation Using Industrial Raw Material

The second validation trial was carried out using the industrial Glucose Syrup (GS 74% Solid Content, 95 DE (Dextrose equivalent). This substrate is used for the manufacturing of $B_2$ using *B. subtilis.*

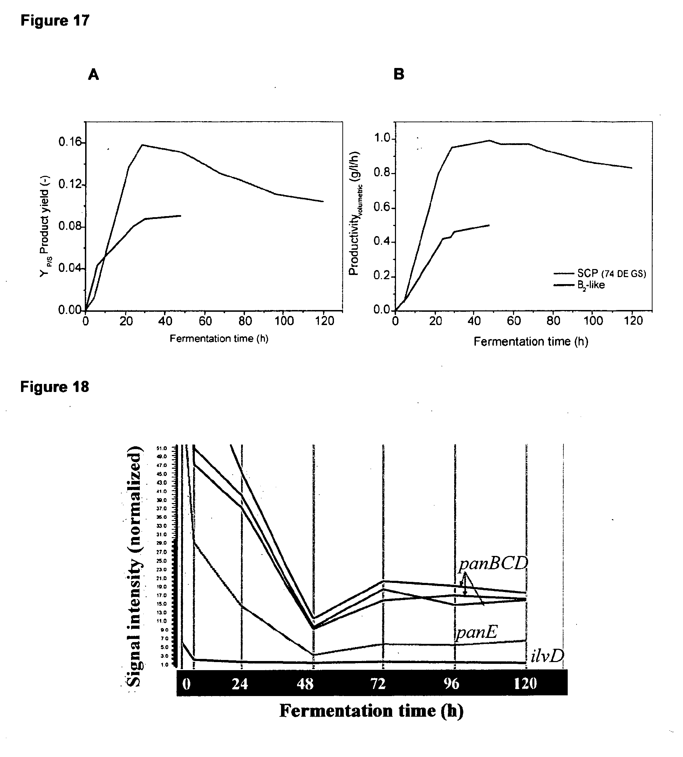

As shown in Fig. 16, *B. subtilis* cultivation could again be extended beyond the standard 48 h, this time showing pantothenate titers up to 49 g/l. As in the previous run, the biomass development showed similar pattern as the pantothenate but with higher final average density (~100 g/l) than reported above. The fermentation time was stopped at 120 h due to shortage in GS. Moreover, due to the higher viscosity of GS and our setting limitation in keeping high temperature in the feeding tank, the feeding rate was somehow lower than the actual setting. As a result of it, the cumulative feeding solution was lower by 12% (11,426g vs. 12,896g at 120h in the previous run). However, the faster reaction and higher yields (Fig. 17) seem to have contributed to achieve comparable pantothenate output as in the previous example. The 751g of pantothenate achieved at the end of this process (Fig. 16B) resembles the -736g achieved at the same time point (120h) in Fig. 14. The positive impact of glucose syrup (GS) on bacterial growth and pantothenate as observed here was not yet a matter of research, it could only be inferred that such an effect is coming, in one hand, from the mixture of dextrose, fructose, maltose, maltotriose and higher saccharides that is known to be present in the syrup. On the other hand, the presence of other macro- as well micronutrients may have been playing an inducer role as well. Certainly, further investigation in this area is required.

Microarray analysis showed that overexpression of the pan genes continued after 48h keeping an upward trend (Fig. 18). From ~72 h towards the end of the process the genes expression levels were maintained within the same range. *ilvD* is depicted in the same figure for reference purposes only. This gene was indeed not engineered and therefore shows a low and flat expression level over the whole fermentation process.

Another interesting observation derived from the application of SCP for pantothenate production is the profile of pantothenate-related-metabolites (PRM) (Fig. 19). From all the PRM, HMBPA is the metabolite that accumulates at the highest level when using standard fed-batch. In SCP although an initial uptrend is observed, it diminishes over the time by a factor of ~75% (Fig. 19A). Similar profiles have been observed for the other PRMs resulting in overall reduction higher than 50% (Fig. 19B). Figure 19B shows also that standard fed-batch operation has indeed the opposite effect. The reduction of PRM will have an important impact on the downstream operations. PRM separation from pantothenate is particularly difficult due to their similar physico-chemical properties (Figure 1 b).

## Claims

1. Method for producing pantothenate by a semi-continuous fermentation process, wherein

   a) the operation time of the process is more than 48 hours;
   b) the feeding rate and profile are set within a range for the bacterial growth rate ($\mu$) that maximizes the conversion efficiency ($Y_{P/S}$) and specific productivity ($q_{P/X}$), and
   c) fermented medium is withdrawn at different times during the fermentation process.

2. Method according to claim 1, wherein the replacement of withdrawn fermented medium by fresh medium is performed by continuous feeding, and wherein the continuous amount of feeding solution (l/h) is adjusted to replace the amount of withdrawn medium until the next medium withdrawal is performed.

3. Method according to any of the preceding claims, wherein the operation time of the fermentation process is more than 60 hours.

4. Method according to any of the preceding claims, wherein the medium withdrawal is performed more than 2 times during the fermentation process.

5. Method according to any of the preceding claims, wherein the medium withdrawal is performed in intervals of less than 30 hours.

6. Method according to any of the preceding claims, wherein the first medium withdrawal is not performed before 6 hours of operation time.

7. Method according to any of the preceding claims, wherein the maximum filling ($V_R^{max.}$) of the fermentation tank is at least 1.1 fold the volume of the initial starting volume.

8. Method according to any of the preceding claims, wherein the feeding solution is reformulated after 48 hours to comprise a higher $PO_4^{3-}$ ion concentration.

9. Method according to claim 8, wherein the reformulated feeding solution comprises a concentration of $PO_4^{3-}$ ions to keep the $PO_4^{3-}$ ion concentration in the fermentation medium at a level of 500 mg/l or higher.

10. Method according to claim 8 or 9, wherein the reformulated feeding solution comprises the following ingredients at a minimum concentration of: $KH_2PO_4$, 6 g/l; $Na_2HPO_4 \cdot 12H_2O$, 10 g/l; $K_2HPO_4$, 6 g/l.

**11.** Method according to any of the preceding claims, wherein the dissolved oxygen ($pO_2$) level is kept at $\geq$ 15%.

**12.** Method according to any of the preceding claims, wherein the feeding rate ranges from 4 g/l/h to 41 g/l/h.

**13.** Method according to any of the preceding claims, wherein the pantothenate is produced at a level greater than 34 g/l before the first withdrawal of medium is performed.

**14.** Method according to any of the preceding claims, wherein the ratio of the initial starting volume in the fermentation tank/ total amount of withdrawn medium is in the range of 1:1 - 1:2.

**15.** Method according to any of the preceding claims, wherein each withdrawal of medium comprises to remove 5%-20% of the fermenter working volume at the time of the medium withdrawal during the fermentation process.

**16.** Method according to any of the preceding claims, wherein the bacterial growth rate ($\mu$) is between 0.03 h$^{-1}$ and 0.07 h$^{-1}$.

**Figure 1a**. Metabolic Pathway of Pantothenate synthesis

**Figure 1b.** Possible formation of pantothenate related metabolites (PRM)

**Figure 2**

**Figure 3**

**Figure 4**

**Figure 5**

**Figure 6**

**Figure 7**

**Figure 8, 9 and 10**

**Figure 11**

**Figure 12**

**Figure 13**

**Figure 14**

**Figure 15**

A

B

**Figure 16**

A

B

**Figure 17**

**A**

**B**

**Figure 18**

**Figure 19**

**A**

**B**

**European Patent Office**

**EUROPEAN SEARCH REPORT**

Application Number

EP 06 02 2822

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2005/103274 A (DSM IP ASSETS BV [NL]; EISELE FRANK [CH]) 3 November 2005 (2005-11-03) * page 4, paragraph 3 * * page 7 - page 8; example 1 * | 1 | INV. C12P13/02 |
| Y | | 2,3,11 | |
| Y,D | WO 01/21772 A2 (OMNIGENE BIOPRODUCTS [US] BASF AG [DE]) 29 March 2001 (2001-03-29) * page 22, line 25 - page 23, line 29 * * page 30, line 8 - line 24 * * page 88, line 25 - page 91, line 20; example XI; tables 22,23 * | 2,3,11 | |
| A | | 13 | |
| Y | WO 2005/017172 A (DSM IP ASSETS BV [NL]; BERRY ALAN [US]; LEE CONNIE [FR]; MAYER ANNE FR) 24 February 2005 (2005-02-24) * page 3, line 34 - page 4, line 11 * * page 4, line 23 - line 29 * * page 5, line 18 - line 23 * * page 6, line 5 - line 9 * * page 6, line 35 - page 7, line 4 * | 2,3 | |
| A | | 4-7,12, 14-16 | TECHNICAL FIELDS SEARCHED (IPC) C12P |
| A | EP 0 796 916 A (TRIPLE A B V [NL]) 24 September 1997 (1997-09-24) * page 2, line 1 - line 50 * * page 3, line 24 - line 28 * * page 4, line 34 - line 39 * | 8-10 | |

The present search report has been drawn up for all claims

| Place of search | Date of completion of the search | Examiner |
|---|---|---|
| Berlin | 22 May 2007 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 02 2822

This annex lists the patent family members relating to the patent documents cited in the above-mentioned European search report.
The members are as contained in the European Patent Office EDP file on
The European Patent Office is in no way liable for these particulars which are merely given for the purpose of information.

22-05-2007

| Patent document cited in search report | | Publication date | Patent family member(s) | | Publication date |
|---|---|---|---|---|---|
| WO 2005103274 | A | 03-11-2005 | KR 20070001244 | A | 03-01-2007 |
| WO 0121772 | A2 | 29-03-2001 | AU 7708700 | A | 24-04-2001 |
| | | | BR 0014115 | A | 21-05-2002 |
| | | | CA 2385497 | A1 | 29-03-2001 |
| | | | CN 1420931 | A | 28-05-2003 |
| | | | EP 1214420 | A2 | 19-06-2002 |
| | | | HU 0202705 | A2 | 28-12-2002 |
| | | | JP 2003527828 | T | 24-09-2003 |
| | | | MX PA02003017 | A | 06-09-2004 |
| | | | NO 20021382 | A | 16-05-2002 |
| | | | PL 356566 | A1 | 28-06-2004 |
| | | | SK 5222002 | A3 | 04-03-2003 |
| | | | TR 200200751 | T2 | 23-08-2004 |
| WO 2005017172 | A | 24-02-2005 | BR PI0413542 | A | 17-10-2006 |
| | | | WO 2005017159 | A2 | 24-02-2005 |
| | | | CN 1882691 | A | 20-12-2006 |
| | | | JP 2007502102 | T | 08-02-2007 |
| | | | KR 20060064629 | A | 13-06-2006 |
| EP 0796916 | A | 24-09-1997 | NONE | | |

EPO FORM P0459

For more details about this annex : see Official Journal of the European Patent Office, No. 12/82

**REFERENCES CITED IN THE DESCRIPTION**

**Patent documents cited in the description**

- WO 0121772 A
- WO 2004005525 A
- US 60262995 B
- US 20050124030 A1
- US 20050124030 A
- WO 2005103274 A

**Non-patent literature cited in the description**

- Biochemical engineering fundamentals. McGraw Hill International Editions, 397
- **GONZÁLEZ-PASTOR et al.** *Science,* 2003, vol. 301, 510-513
- **LEE et al.** *J. Bacteriol,* 2001, vol. 183, 7371-7380